# EUROPÄISCHE PATENTSCHRIFT

(11) **EP 0 312 617 B1**
(45) Veröffentlichungstag und Bekanntmachung des Hinweises auf die Patenterteilung: **03.03.1993**
(21) Anmeldenummer: 88904712.2
(22) Anmeldetag: 14.04.1988
(51) Int. Cl.: C12P 21/02, C12N 15/00

(54) **VERFAHREN ZUR HERSTELLUNG VON POLYPEPTIDEN IN ZELLFREIEN TRANSLATIONSSYSTEMEN**
METHOD OF OBTAINING POLYPEPTIDES IN CELL-FREE TRANSLATION SYSTEM
PROCEDE POUR OBTENIR DES POLYPEPTIDES DANS UN SYSTEME DE TRANSLATION ACELLULAIRE

(30) Priorität: 29.04.1987 SU 4239148
(43) Veröffentlichungstag der Anmeldung: 26.04.1989
(73) Patentinhaber: INSTITUT BELKA AKADEMII NAUK SSSR, Puschino 142292 (RU)
(72) Erfinder: ALAKHOV, July Borisovich, Puschino, 142292 (SU); BARANOV, Vladimir Ivanovich, Puschino, 142292 (SU); OVODOV, Sergei Jurievich, Puschino, 142292 (SU); RYABOVA, Ljubov Anatolievna, Puschino, 142292 (SU); SPIRIN, Alexandr Sergeevich, Moscow, 117420 (SU)
(74) Vertreter: von Füner, Alexander, Dr.
(86) Internationale Anmeldenummer: SU8800078
(87) Internationale Veröffentlichungsnummer: WO8808453

(56) Entgegenhaltungen:
- EP-A- 0 152 483
- CHEMICAL ABSTRACTS, Band 97, Nr. 25, 20. Dezember 1982, Seiten 340-341, Zusammenfassung Nr. 210628q, Columbus, Ohio, US; S. WANG et al.: "Translation of poly(A) RNA from rat liver in wheat germ cell-free system", & SHENGU'U HUAXUE YU SHENGU'U WULI JINZHAN 1982, 46, 37-40
- CHEMICAL ABSTRACTS, Band 97, Nr. 25, 20. Dezember 1982, Seite 341, Zusammenfassung Nr. 210629r, Columbus, Ohio, US; G.WU et al.: "Translation of mammalian messenger RNA in cell-free protein-synthesizing system derived from rabbit reticulocyte lysate", & SHENGU'U HUAXUE YU SHENGU'U WULI JINZHAN 1982, 46, 52-6
- SCIENCE, Band 242, 25. November 1988, Seiten 1101-1216, American Association for the Advancement of Science; A.S. SPIRIN et al.: "A continuous cell-free translation system capable of producing polypeptides in high yield"
- Proceedings of the National Academy of Sciences of the United States of America, vol. 82, Nr. 15, August 1985 (Washington) K.E. Rogers et al "Olfactory neuron-specific protein is translated from a large poly (A)+ mRNA", see pages 5218-5222
- Proceedings of the national Acvademy of Sciences of the United States of America, vol. 82, Nr. 6, March 1985 (Washington) M. Clelia Ganoza et al "Isolation and point of action of a factor from Escherichia coli reguired to reconstruct translation", see pages 1648-1652
- European Journal of Biochemistry, vol. 93, Nr. 1, January 1979, (published by Springer-Verlag Berlin Heidelberg New York), Warwich Bottomley et al "Cell-free transcription and translation of total spinach chloroplast DNA", see pages 31-39
- Biopolimery I Kletka, vol. 4, Nr. 3, May-June 1988 (Naukova dumka Kiev), A.P. Potapov et al, see pages 133-138

## Beschreibung

### Gebiet der Technik

Die vorliegende Erfindung bezieht sich auf die Molekularbiologie und Biotechnologie und betrifft insbesondere Verfahren zur Herstellung von Polypeptiden in einem zellfreien Translation-System.

Die genannten Polypeptide werden umfassend in der Medizin als Bioregulatoren der biologischen Prozesse verwendet. Beispeilsweise, sind Polypeptide-Aktivatoren des Immunosystems, Polypeptide-Neuromediatoren und Trasmitter, Polypeptide-Regulatoren des Salzstoffwechsels und anderes mehr bekannt. Bekannt ist die Verwendung der Polypeptide in der Landwirtschaft als Biostimulatoren, beispielsweise Wachstumshormone. Polypeptide werden auch in der Bioelektronik, beispielsweise als Filme mit Rhodopsin verwendet.

### Vorhergehender Stand der Technik

Bekannt sind zwei Methoden zur Herstellung von Polypeptiden: chemische Synthese und }Methode der Gentechnik.

Die Methode der chemischen Synthese wird umfassend bei der großtechnischen Produktion von Polypeptiden mit relativ geringer Länge (höchstens 15 Aminosäurereste) und nur in Ausnahmefällen für die Synthese einiger Polypeptide größerer Länge (25-30 Aminosäurereste) angewendet. Die Produktion von Polypeptiden größerer Länge unter Verwendung dieser Methode ist praktisch unzugänglich. Das ist darauf zurückzuführen, daß bei der Anwendung der Methode der chemischen Synthese mit der Vergrößerung der Polypeptidkette die Ausbeute an Endprodukt infolge der Razemisierung der Aminosäurereste, der Unvollständigkeit,der Verlängerung der Polypeptidkette, der Kompliziertheit der Wahl von Schutzgruppen und ihrer Beseitigung exponential herabsinkt. All das verursacht große Schwierigkeiten bei der Reinigung des Endproduktes und eine starke Steigerung der Kosten der Polypeptide im Maße der Vergrößerung ihrer Länge.

Die Methode der Gentechnik für die präparative Speicherung von Polypeptiden ist auch in der Anwendung begrenzt. Das ist mit der Kompliziertheit der Aussonderung des Expressionsproduktes mit Hilfe der transformierten Zellen, mit der Letalität einiger Endprodukte für die Produzenten-Zelle, mit der Ausscheidung der transformierten Plasmide aus einer Zelle und mit der proteolytischen Degradation des Expressionsproduktes eines fremden Gens verbunden. Der letzte Umstand ruft besondere Schwierigkeit bei der Aussonderung vieler Polypeptide, insbesondere Polypeptide mit einer Länge von 15 bis 70 Aminosäureresten hervor, die sich infolge des Fehlens kompakter Struktur in ihnen durch die intra- und extrazellulären Porteasen abbauen lassen.

Aus dem Obendargelegten geht hervor, daß die beschriebenen Methoden der Produktion von Polypeptiden für die Erzeugung der Polypeptide mit einer Länge von 15 bis 70 Aminosäureresten praktisch nicht anwendbar sind. Zugleich ist es bekannt, daß gerade solche Länge viele biologisch aktive Polypeptide, solche wie Peptide-Aktivatoren des Immunsystems, Peptide-Neuromediatoren und Transmitter sowie Peptide-Aktivatoren des Salzstoffwechsels aufweisen.

Es besteht eine weitere Methode zur Synthese von Polypeptiden, die auf der Nutzung eines zellfreien Translation-Systems beruht. Gegenwärtig ist eine ganze Reihe von zellfreien Translation-Systemen auf der Grundlage von Zellenextrakten aus verschiedenen prokaryonten und eukaryonten Organismen entwickelt.

Bekannt ist, beispielsweise, ein Verfahren zur Erzeugung von Polypeptiden, das darin besteht, daß man in einem zellfreien Translation-System aus Weizenkreimen die Synthese des Globin-Polypeptids eines Kaninchens vornimmt (Proc. Nat. Acad. Sci. USA. 70 1973 USA).

1 ml zellfreies Translation-System an Ribosomen enthält 0,5 ml Extrakt aus Weizenkeinem, 0, 125 nMol 9S mRNS des Kaninchensglobins, 8 mMol Kreatinphosphat, 40 g Kreatinphosphokinase, 2-10 µg Ribonuklease-Inhibitor aus der Plazenta des Menschen, je 0,1 µg Protease-Inhibitoren (Pepstatin, Chymostatin, Antipain, Leupeptin) in einer Pufferlösung von 20 mMol HEPES (pH 7,6), die 80 mMol K⁺ - Azetat, 3,0 mMol Mg²⁺ Azetat, 1 mMol ATP, 20 mMol GTP, 2 mMol Dithiotreit, 100 µCi/mMol [³⁵S]-Methionin (spezifische Aktivität beträgt 89 Ci/mMol, 19 übrige Aminosäuren zu je 20 mMol jede enthält.

Die Synthese des Polypeptids in einem zellfreien Translation-System wird bei einer Temperatur von 25°C durchgeführt. Bei der Synthese werden im System Translationsprodukte gespeichert, die das Endprodukt und die Abbauprodukte in Form von AMP, GDP, Pyrophosphat, Phosphat, Kreatin u.a. aufweisen. Infolge der Inhibierung wird die Synthese des Endproduktes in 60 bis 90 Minuten zum Stillstand gebracht. Die Analyse des Endproduktes nach einer radioaktiven Markierung [³⁵S]-Methionin, die in das Polypeptid einbezogen ist, erfolgt im Verfahren der heißen Fällung von Polypeptid an einem Filter mit 5%iger Trichloressigsäure. Die Menge des synthetisierten Endproduktes beträgt in 60 bis 90 Minuten < 0,58 Picomol Globins je 1 Picomol der Matrix.

Des obenbetrachtete Verfahren zur Herstellung von Polypeptiden ist eines der von den bekannten besonders effektiven Verfahren, in denen zellfreie Translationssysteme verwendet werden. Sogar diese Systeme gewährleisten jedoch die Synthese maximal nur von einer oder zwei Kopien des Polypeptids je eine Kopie der mRNS infolge einer kurzen Dauer der Arbeit eines derartigen zellfreien Systems, lediglich 1 bis 2 Stunden.

Die Hauptursachen der kurzen Dauer der Arbeit der zellfreien Translation-Systeme bestehen in folgendem:
1. Zellfreie Translation-Systeme enthalten eine begrenzte Anzahl von ATP- und GTP-Energieträgern, die Erhöhung des Gehaltes an denen zur Inhibierung des jeweiligen Translation-Systems führt.
2. Die Abbauprodukte, die bei der Synthese des Polypeptids in einem zellfreien Translation-Systems ADP, AMP, GDP, Phosphate und Pyrophosphate entstehen, sind Inhibitoren der Synthese von Polypeptiden.
3. Endprodukt kann auch Inhibitor der Synthese sein.

### Offenbarung der Erdindung

Der Erfindung liegt die Aufgabe zugrunde, ein solches Verfahren zur Herstellung von Polypeptiden in einem zellfreien Translation-System zu entwickeln, welches es ermöglicht, ein Endprodukt in präparativen Mengen durch die Vergrößerung der Dauer der Arbeit des zellfreien Systems der Synthese von Polypeptiden zu gewinnen.

Diese Aufgabe wird dadurch gelöst, daß ein solches Verfahren zur Herstellung von Polypeptiden in einem zellfreien Translation-System an Ribosomen, das als Substrate ATP, GTP und Aminosäuren enthält, unter Entstehung von Translationsprodukten im System, die das Endprodukt, AMP, GDP, Pyrophosphat und anorganisches Phosphat aufweisen, vorgeschlagen wird, in dem erfindungsgemäß bei der Translation im Maße des Verbrauchs der Substrate mit Anfallen der Produkte aus dem System die Produkte der Translation, die AMP, GDP, Pyrophosphat, anorganisches Phosphat und Endprodukt aufweisen, mit der gleichzeitigen Einführung von Substraten ins System in Form von Aminosaüren, ATP und GTP für die Unterhaltung ihrer Ausgangskonzentration herausgeführt werden.

Als ein zellfreies Translation-System können in dem erfindungsgemäßen Verfahren prokaryonte beziehungsweise eukaryonte zellfreie Translation-Systeme verwendet werden, die sowohl endogene als auch exogene natürliche oder künstlich synthetisierte mRNS aufweisen. In dem erfindungsgemäßen Verfahren werden jene Verhältnisse der Komponenten im Reaktinsgemisch, Ionen- und Temperaturbedingungen der Synthese angewendet, die für das gewählte zellfreie Translation-System optimal sind.

Die erfindungsgemäße Methode der Synthese von Polypeptiden in einem zellfreien Translation-System ist frei von den Nachteilen der Methoden der chemischen Synthese und der Gentechnik und kann für die Herstellung von Polypeptiden beliebiger Länge und Aminosäure-Konsequenz eingesetzt werden. Diese Eigenschaft ist besonders für die Herstellung von Polypeptiden mit einer Länge von 15 bis 70 Aminosäureresten von Bedeutung, deren industriemäßige Herstellung in anderen Verfahren gegenwärtig unzugänglich ist oder einen äußerst konstspieligen Prozeß darstellt.

Das erfindungsgemäße Verfahren bewirkt die Synthese von Polypeptiden in einem zellfreien Translation-System mit einer konstanten Geschwindigkeit innerhalb von 40 Stunden und darüber hinaus. Die Menge des gewonnenen Endproduktes beträgt dabei 200 bis 300 Kopien des Polypeptids je eine mRNS-Kopie. Aus 1 Liter des Reaktionsgemisches gelingt es bei der Anwendung des erfindungsgemäßen Verfahrens, bis zu 100 mg Fertigproduktes innerhalb von 50 bis 100 Stunden der Arbeit des Systems zu gewinnen. Das erschließt Möglichkeiten für die industriemäßige Produktion von Polypeptiden.

### Kurze Beschreibung der Zeichnungen

Nachstehend wird die Erfindung anhand der Beispiele ihrer Ausführung mit Bezugnahme auf beigefügte Zeichnungen erläutert, in denen es zeigen:
Fig. 1, 2, 3 und 4 - Diagramm der Abhängigkeit der Menge des zu synthetisierenden Polypeptids je mRNS-Einheit von der Synthesedauer;
Fig. 5 stellt ein Bild des SDS-Harnstoff-Polyakrylamid-Gels dar, an dem die Verteilung der gewonnenen Polypeptide entsprechend ihrem Molekulargewicht gezeigt wird.

### Beste Ausführungsvariante der Erfindung

Das Verfahren zur Herstellung von Polypeptiden in einem zellfreien Translation-System ist einfach in technologischer Ausführung und wird folgt durchgeführt.

Das zellfreie Translation-System aus Prokaryonten oder aus Eukaryonten, das Ribosome, Faktoren der Translation, tRNS-, mRNS-Aminoazyl, Substrate der Reaktion, einschließlich ATP, GTP und Aminosaüren aufweist, wird in an sich bekannter Weise hergestellt.

Das zellfreie Translation-System unterbringt man in eine Standard-Zelle zur Ultrafiltration, die mit einer semipermeablen Membrane mit einem Porendurchmesser versehen ist, der für die Permeabilität des Endproduktes durch die Membrane ausreichend ist. Der Inhalt der Zelle wird dann in einem Thermostat auf die erforderliche Temperatur erwärmt.

Bei der Synthese wird aus der Zelle durch die semi-permeable Membrane das Abpumpen der Translationsprodukte aus dem System, einschließlich Abbauprodukte und das synthetisierte Endprodukt, vorgenommen. Gleichzeitig damit werden dem System Substrate in Form von ATP, GTP und Aminosäuren zur Unterchaltung ihrer Ausgangskonzentration aus einem Extrabehälter zugeführt.

Zur weiteren Ausscheidung und Reiningung des Endproduktes wird die aus dem System abzupumpende Lösung einer Säule mit einem Immunaffinitätssorptionsmittel zugeführt, an dem die selektive Absorption des Endproduktes erfolgt. Nach der Beendigung der Synthese wird das Endprodukt von der Säule mit dem Immunosorptionsmittel abgespült und entsalzt.

### Beispiel 1

1 ml zellfreies Translation-System enthält 0,6 nMol 70S Ribosome E.coli, 1 mg Fraktion S 100, 0,6 mg tRNS, 0,06 nMol mRNS des Proteins der Hülle der MS2-Phage, 5 µg Pyruvatkinase, 2 bis 10 µg Ribonuklease-Inhibitor aus der Plazenta des Menschen, je 0,1 µg Protease-Inhibitoren (Aprotinin, Leupeptin, Chymostatin) in einer Pufferlösung: 20 mMol Tris HCl (pH 7,4), 100 mMol NH₄Cl, 10 mMol MgCl₂, 1 mMol ATP, 0,2 mMol GTP, 5 mMol Phosphoenolpyruvat, 25 mMol [³H]⁻-Leuzin (spezifische Aktivität 52 Ci/mMol) und je 25 mMol übrige 19 Aminosäuren.

Das zellfreie Translation-System unterbringt man in einer Zelle für Ultrafiltration und führt man die Synthese des Polypeptids bei einer Temperatur von 37°C durch. Die Wahl der Produkte der Translation, die das Endprodukt und die Abbauprodukte aufweisen, erfolgt durch eine semipermeable Membrane mit der gleichzeitigen Zuführung von Substraten in Form von ATP, GTP und Aminosäuren dem Reaktionsgemisch innerhalb von 20 Stunden. Hierdurch erhält man Protein der Hülle der MS2-Phage.

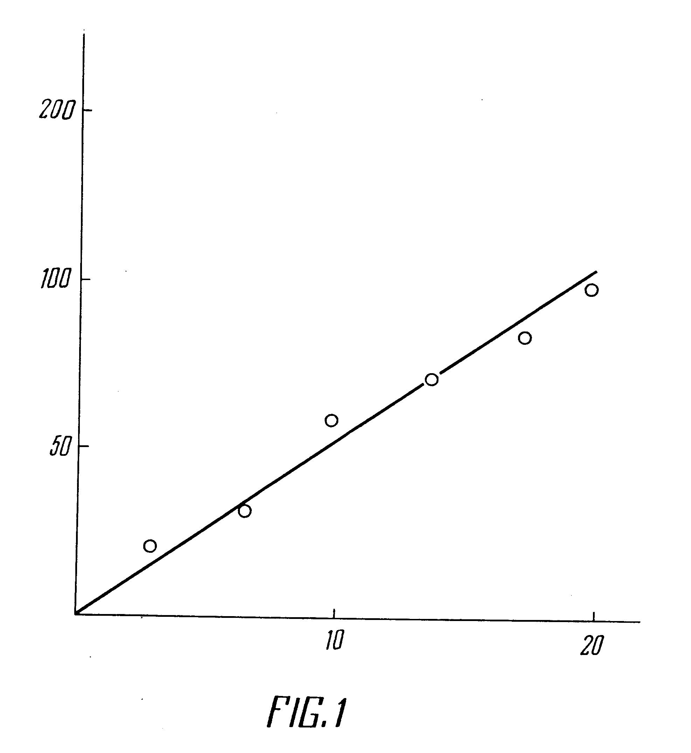

Während dieser ganzen Zeit erfolgt die Synthese des Endproduktes mit konstanter Geschwindigkeit. Die Abhängigkeit der Menge des herzustellenden Proteins der Hülle je mRNS-Einheit von der Dauer der Synthese ist in der Fig. 1 angeführt. An der Abszissenachse ist die Dauer der Synthese in Stunden und an der Ordinatenachse die Menge des anfallenden Produktes in Picomol abgelegt.

Hierdurch wurden im Verlaufe der Arbeit des zellfreien Translation-Systems aus E.coli 6000 Picomol des Hüllenproteins gewonnen, was 100 Picomol des Endproduktes je 1 Picomol der mRNS der Hülle der MS2-Phage betrug.

### Beispiel 2

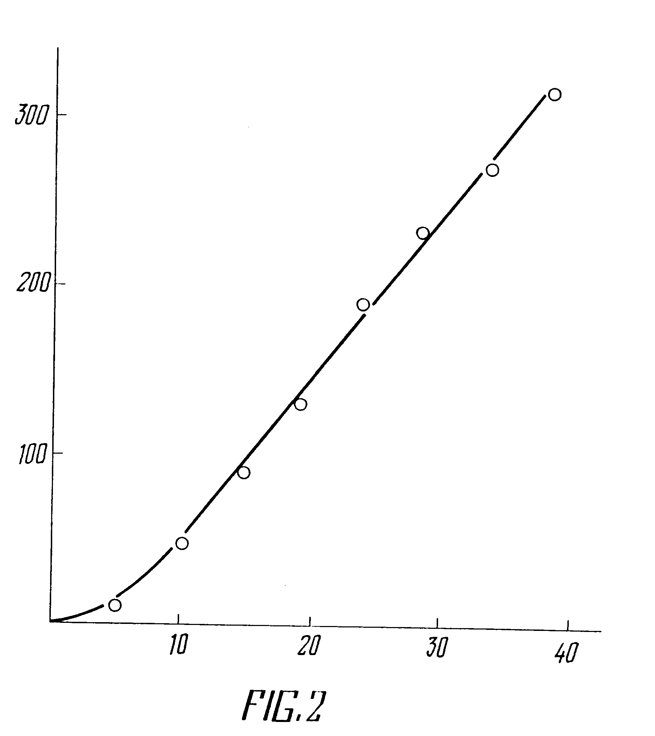

Man gewinnt Kalzitonin nach der in Beispiel, 1 beschriebenen Methodik mit Ausnahme dessen, daß man anstelle des mRNS-Proteins der Hülle der MS2-Phage 0,06 mMol des mRNS-Kalzitonins verwendet. Hierdurch erhält man Kalzitonin-Peptid. Während der ganzen Zeit geht die Synthese des Endproduktes mit konstanter Geschwindigkeit vor sich. Die Abhängigkeit der Menge des angallenden Kalzitonins je mRNS-Einheit von der Dauer der Synthese ist in der Fig. 2 abgebildet. Hierdurch wurden während der Arbeit des zellfreien Translation-Systems aus E.coli 18000 Picomol Kalzitonin gewonnen, was 300 Picomol Fertigproducktes je 1 pMol des mRNS-Kalzitonins beträgt.

### Beispiel 3

1 ml zellfreies Translation-System enthält 0,5 ml Extrakt aus Weizenkeimen, 0,1 mMol mRNS-Virus BMV, 64 µg Kreationsphosphokinase, von 2 bis 10 µg Ribonuklease-Inhibitor aus der Plazenta des Menschen, je 0,1 µg Protease-Inhibitor (Aprotinin, Pepstatin, Leupeptin) in einer Pufferlösung: 40 mMol HEPES (pH 7,6), 112 mMol K⁺ Azetat, 1,9 mMol Mg²⁺ Azetat, 0,25 mMol Spermidin, 6 mMol Dithiotreit, 1,5% Glyzerin, 2 mMol ATP, 50 mMol GTP, 8 mMol Kreationsphosphat, 25 Mol [³H] -Leuzin (spezifische Aktivität 50 Ci/mMol) und je 25 mMol übrige 19 Aminosäuren.

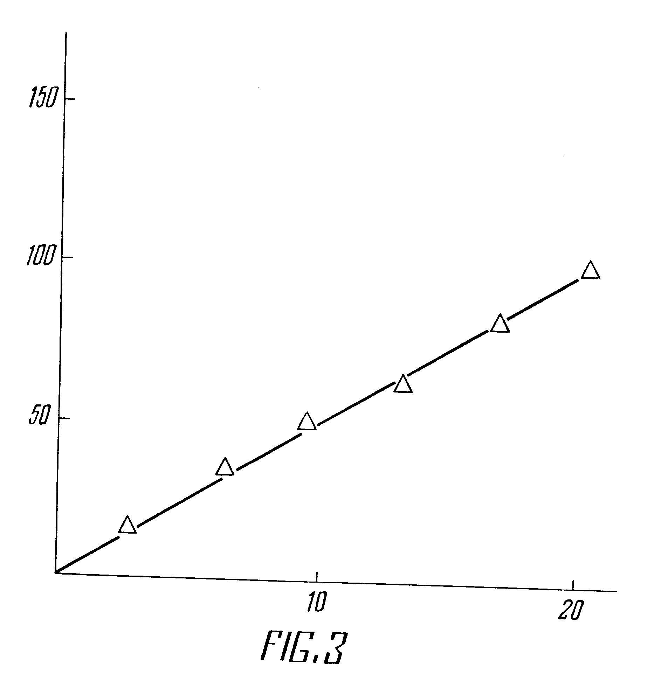

Das zellfreie Translation-System unterbringt man in eine Zelle für die Ultrafiltration und man führt die Synthese der Polypeptide bei einer Temperatur von 25°C durch. Die Wahl der Translationsprodukte, die das Endprodukt und die Abbauprodukte enthalten, erfolgt durch eine semipermeable Membrane mit der gleichzeitigen Zuführung von Substraten in Form von ATP, GTP und Aminosäuren innerhalb von 20 Stunden. Im Ergebnis erhält man Protein der Hülle des BMV-Virus. Während der ganzen Zeit erfolgt die Synthese des Endproduktes mit einer konstanten Geschwindigkeit. Die Abhängigkeit der Menge des anfallenden Proteins der Hülle des BMV-Virus je mRNS-Einheit von der Dauer der Synthese ist in der Fig. 3 abgebildet. Hierdurch wurden während der Arbeit des zellfreien Translation-Systems aus Weizenkeimen 1000 Picomol Protein der Hülle des BMV-Virus gewonnen, was 100 Picomol Fertigprodukt je 1 Picomol mRNS des Proteins der Hülle des BMV-Virus betrug.

### Beispiel 4

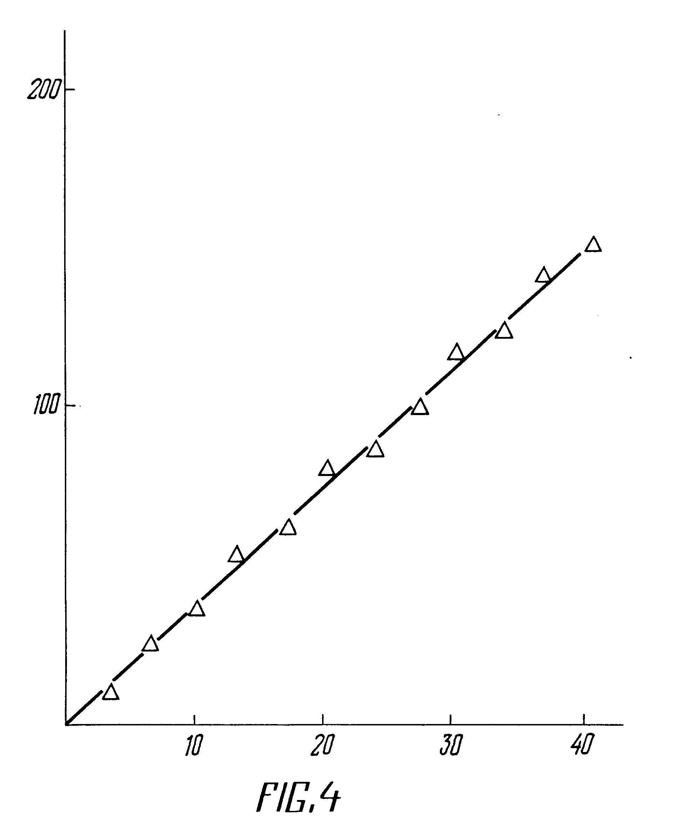

Man erhält das Kalzitonin nach der in Beispiel 3 beschriebenen Methodik mit Ausnahme dessen, daß man anstelle von mRNS-Kalzitonin verwendet. Hierdurch erhält man Kalzitonin-Peptid. Während der ganzen Zeit erfolgt die Synthese des Endproduktes mit konstanter Geschwindigkeit. Das Diagramm der Abhängigkeit der Menge des anfallenden Kalzitonins je Einheit des mRNS-Kalzitonins von der Dauer des Synthese ist in Fig. 4 abgebildet. Hierdurch wurden während der Synthese im zellfreien Translation-System aus Weizenkeimen 15000 Picomol Kalzitonin gewonnen, was 150 Picomol Fertigproduktes je 1 Picomol mRNS-Kalzitonins betrug.

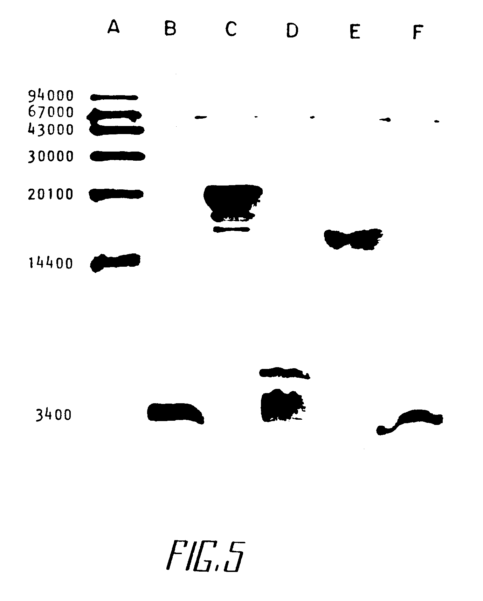

Elektrophoretische Analyse der gewonnenen Polypeptide (Beispiele 1 bis 4) ist in der Fig. 5 abgebildet. Die Färbung der Polypeptide im Gel erfolgt mit Goomassie brilliant blue G-250
A - Standardsatz von Polypeptiden (Amersham)
B - kommerzielles Kalzitonin (Salmon calcitonine, Sigma)
C - Protein der Hülle des BMV-Virus (Beispiel 3)
D - Kalzitonin: (Beispiel 4) der untere Striefen - Kalzitonin mit Molekulargewicht von 3400; der obere Streifen - Beimengung der dimeren Form des Kalzitonins;
E - Protein der Hülle der MS2-Phage (Beispiel 1);
F - Kalzitonin (Beispiel 2).

Aus der vorgelegten Fotografie ist zu ersehen, daß die anfallenden Polypeptide ein Molekulargewicht aufweisen, das ihren Naturanalogen entspricht.

### Industrielle Anwendbarkeit

Die gemäß dem vorgeschlagenen Verfahren herzustellenden Polypeptide können in der Medizin, Landwirtschaft und in der Bioelektronik verwendet werden.

## Patentansprüche

1. Verfahren zur Herstellung von Polypeptiden in einem zellfreien Translations-System an Ribosomen, das als Substrate ATP, GTP und Aminosäuren aufweist, mit Enstehung von Translationsprodukten im System, welches die Endprodukte, AMP, GDP, Pyrophosphat und anorganisches Phosphat beinhaltet, dadurch gekennzeichnet, daß während der Translation im Maße des Verbrauchs der Substrate und der Bildung der Produkte der Translation, AMP, GDP, Pyrophosphat, anorganisches Phosphat und Endprodukte herausgeführt werden und gleichzeitig Substrate, nämlich Aminosäuren, ATP und GTP zur Erhaltung ihrer Ausgangskonzentration eingeführt werden.

## Claims

1. A method of preparing polypeptides in a cell-free translation system on ribosomes, containing ATP, GTP and amino acids as the substrates, with formation of the translation products in the system including the endproducts, AMP, GDP, pyrophosphate and inorganic phosphate, characterized in that in the course of the translation process, as the substrates are consumed and the products of translation are obtained, AMP, GDP, pyrophosphate, inorganic phosphate and the desired products are extracted, while simultaneously introducing substrates in the form of amino acids, ATP and GTP for the purpose of maintaining their initial concentration.

## Revendications

1. Procédé d'obtention de polypeptides dans un système de translation acellulaire sur des ribosomes, qui en tant qui substrat comporte de l'ATP, du GTP et des amino-acides, avec production dans le système de produits de translation, qui consistent en les produits finaux, AMP, GAP, pyrophosphate et phosphate inorganique, caractérisé en ce que, durant la translation, on recueille, en une proportion qui est fonction de la consommation de substrat et de la formation des produits de la translation, les produits finaux, AMP, GDP, pyrophosphate et phosphate inorganique et en ce que, simultanément, on ajoute du substrat, à savoir, acides aminés, ATP et GTP, pour la conservation de la concentration initiale.
